# EUROPEAN PATENT SPECIFICATION

(11) **EP 0 506 914 B1**
(45) Date of publication and mention of the grant of the patent: **18.12.1996**
(21) Application number: 91918279.0
(22) Date of filing: 11.10.1991
(51) Int. Cl.: G01N 33/573, G01N 33/53, G01N 33/68, G01N 33/569, G01N 33/574, C07K 5/00

(54) **METHOD FOR THE DIAGNOSIS OF HIV INFECTIONS**
VERFAHREN ZUR DIAGNOSE VON HIV-INFEKTIONEN
PROCEDE DE DIAGNOSTIC DES INFECTIONS PAR LE HIV

(30) Priority: 12.10.1990 EP 90402863
(43) Date of publication of application: 07.10.1992
(73) Proprietor: PRO-SOMA SARL, 75005 Paris (FR)
(72) Inventor: SCHERRER, Klaus, F-75005 Paris (FR); BUREAU, Jean-Paul, F-34170 Castelnau-le-Lez (FR); BEY, Fayçal, F-75003 Paris (FR)
(74) Representative: Hermans, Franciscus G.M.
(86) International application number: EP9101946
(87) International publication number: WO9207270

(56) References cited:
- EP-A- 0 219 368
- EP-A- 0 345 750
- RESEARCH REAGENT NEWS, vol. 1, 01 January 1990, Turnhout (BE); ANONYMOUS, pp. 1-4
- "The Theory and Practice of Oncology", R. W. Raven, The Parthenon Publishing Group, pages 285 - 287, 337

### SEQUENCE LISTING

(1) GENERAL INFORMATION:
   (i) APPLICANT:
      (A) NAME: PRO-SOMA SARL
      (B) STREET: 9, Rue Larrey
      (C) CITY: Paris
      (E) COUNTRY: France
      (F) POSTAL CODE: F 75 005
   (ii) TITLE OF INVENTION: Diagnostic Method
   (iii) NUMBER OF SEQUENCES: 1
(2) INFORMATION FOR SEQ ID NO:1:
   (i) SEQUENCE CHARACTERISTICS:
      (A) LENGTH: 4 amino acids
      (B) TYPE: amino acid
      (C) TOPOLOGY: linear
   (ii) MOLECULE TYPE: protein
   (xi) SEQUENCE DESCRIPTION: SEQ ID NO:1:
   Leu Leu Val Tyr
   1

## Description

Technical Field. This invention relates to a diagnostic methods useful in the diagnosis of cancer and HIV infection.

Background Art: European Patent application 219,368 A (corresponding to U. S. Patent Application Serial No. 07/298,791) discloses certain monoclonal antibodies directed against proteins which form constituent parts of the "prosome". Prosomes are intracellular particles described in Schmid et al "The prosome: an ubiquitous morphologically distinct RNP particle associated with repressed mRNPs and containing specific ScRNA and a characteristic set of proteins," The EMBO Journal, 3(1), 29-34 (1984). The prosome has been also variously called the "proteasome" or "mcp" (multicatalytic protease).

Since early 1990, mouse monoclonal antibodies against prosomal proteins have been commercially available from Organon Teknika nv of Turnhout, Belgium. The commercially available monoclonal antibodies have been ones directed against prosomal proteins p23K, p25K, p27K, p29K (p28K, p33K), p30/33K, and p31K and the anti-p21K ("prosome-like particle"). These antibodies are more thoroughly described in Research Reagent News, (Jan. 1990) available from Organon Teknika nv.

In EP 219,368 A1, it is also disclosed that prosomes may be involved in many physiological processes related to the differentiation of cells and organisms, communication between cells, and to autoimmune disease. Also disclosed is that monoclonal antibodies directed against prosomes can be useful in the diagnosis of, for example, cancer.

In Research Reagent News, disclosed uses for the monoclonal antibodies against the prosomal proteins include the study of the molecular biology of prosomes, the distribution of specific types of prosomes in normal and pathological cells and tissues, messenger and other ribonucleoproteins.

The prosome has been also variously called the "proteasome" or "mcp" (multicatalytic protease). European Patent application 0 345 750 A2 discloses a "polyfunctional protease", and antibodies thereto. The polyfunctional protease described in that application may be the prosome.

In EP 0 345 750 the determination of the polyfunctional protease activity from several benignant diseases and malignant diseases (Esophageal cancer, Hepatic cancer, Pulmonary cancer, Ovary cancer and Leukaemia) is described.

No where is it described or suggested that the presence of prosomes outside of the cell might have any possible usefulness in the diagnosis of infectious diseases such as AIDS or HIV infection, or that these extracellular prosomes might have use in the diagnosis of colon cancer.

### Disclosure of the Invention.

Surprisingly, it has been found that persons infected with HIV have increased levels of extracellular prosomes in their blood in comparison to the blood of persons not suffering from this disease. The presence and quantity of these extracellular prosomes can be detected with certain labelled anti-prosomal protein monoclonal antibodies (e.g. monoclonal antibodies directed against prosomal proteins p25K, p27K, p29K (p28K, p33K), p30/33K, and p31K). The invention therefore includes a method of diagnosing HIV infection using these anti-prosomal protein monoclonal antibodies, no matter what the source, to detect and measure the amount of prosomes existing extracellularly in the blood.

Such a method would typically involve: obtaining a sample of blood from a patient suspected of being infected with HIV; extracting the portion of blood which would contain extracellular prosomes; contacting the extracted portion with an immunochemical agent containing one or more of the described monoclonal antibodies; and detecting the presence and relative amount of immune complexes formed between the extracellular prosomes and the monoclonal antibodies. These results once obtained can be compared with a "norm" derived from a person believed not to be suffering with the diseases.

The amount of these extracellular prosomes can also be determined by measuring the amount of protease activity in an extracted portion of blood which would contain these extracellular prosomes. The greater the number of prosomes the greater the amount of protease activity.

A method of diagnosing HIV is also included as part of the invention.

### Brief Description of the Figures

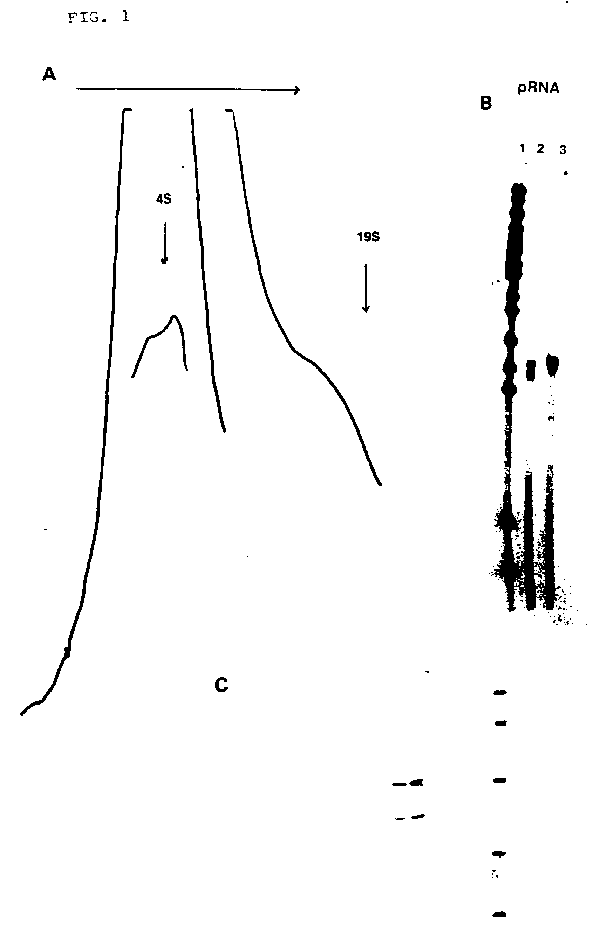

FIG. 1 depicts various panels of EXAMPLE 1:
Panel A depicts prosomes prepared from calf sera loaded onto a 0.5% sarkosyl sucrose gradient (5-25%).
Panel B depicts prosomes from human sera which were 3' end labelled: the RNA's were phenol extracted and analysed on a a 8% acrylamide-urea gel. Column 1 is markers, column 2 contains prosomes from sera; and column 3 contains Hela cell prosomes.
Panel C is a Western blot analysis of gradient fractions with 5 anti-prosome monoclonal antibodies.

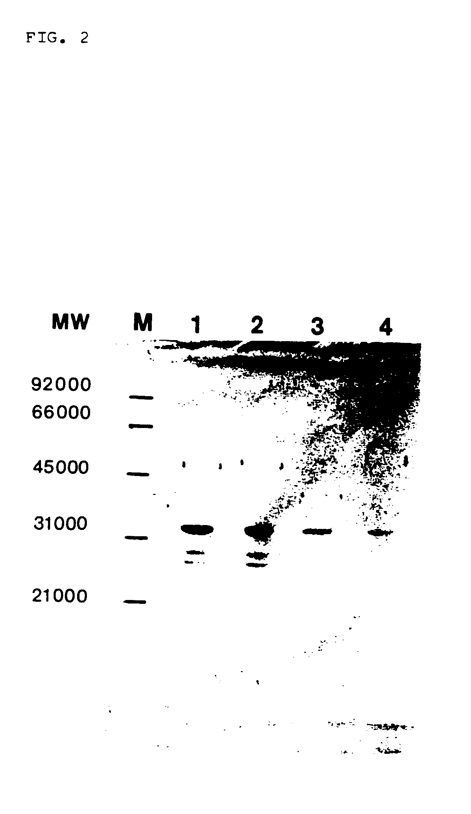

FIG. 2 depicts the increased level of extra cellular prosomes in cancer: Western blot analysis of extra-cellular prosomes isolated from normal human sera and from colon cancer patients (comparative data).

### Best mode of the invention

### I. Antibodies-

Antibodies are preferably used to isolate or identify the extracellular prosomes of the subject to be diagnosed. Such antibodies are preferably monoclonal antibodies or monospecific polyclonal (e.g. affinity purified) antibodies, although polyclonal antibodies and antibody fragments may also be used.

Antibodies against prosomal proteins for use in diagnostic kits and methods according to the invention are preferably monoclonal antibodies directed against the various prosomal proteins. Polyclonal antibodies and modified polyclonal antibodies may also be used however.

Monoclonal antibodies produced against the proteins can be produced by biologically pure cell lines of immortalized antibody-producing cells. Immortalized antibody producing cells can be obtained according to any of the various methods which are known in the art, and generally include the steps: 1) inducing suitable cells such as lymphocytes to produce specific antibodies (for example by injecting an immunogen, such as the polypeptide of FIG. 1 or FIG. 2), 2) immortalizing those cells, and 3) selecting clones out of these cells which produce antibodies of the desired specificity and affinity. For example, one method would be that of Kohler and Millstein, Nature, vol. 256, 495-497 (1975). This method comprises immunizing mice with the particular peptide (e.g. that of FIG. 1 or FIG. 2), isolating spleen cells and fusing these with mouse myeloma cells to obtain hybridomas. Of course animals other than mice could be used as well.

All of the antibodies directed against prosomal proteins specifically identified herein are commercially available from Organon Teknika nv. Deposits of cell lines producing monoclonal antibodies against the p27K and p31K have been made with the Collection Nationale de Cultures des Microorganismes of the Pasteur Institute under numbers I-588 and I-589. The antibodies may be likewise made as described in EP 219,368 A1 (corresponding to U. S. Patent Application Serial No. 07/298,791).

### II. The peptide substrate

The peptides and peptide derivatives according to the general formula: may be prepared in a manner conventional for peptides. For example, the compounds may be synthesized by the solid phase method of Merrifield. Different solid supports and different strategies are known see, e.g. Barany and Merrifield in The Peptides, Analysis, Synthesis, Biology, Vol. 2, E. Gross and J. Meienhofer, eds., (Acad. Press, N.Y., 1980), Kneib-Cordonier and Mullen Int. J. Peptide Protein Res., 30, 705-739 (1987) and Fields and Noble Int. J. Peptide Protein Res., 35, 161-214 (1990).

Removal of the protecting groups, and, in the case of solid phase peptide synthesis, the cleavage from the solid support, can take place in different ways, depending on the nature of any protecting groups and the type of linker to the solid support. Usually deprotection takes place under acidic conditions and in the presence of scavengers. See, e.g. volumes 3, 5 and 9 of the series on The Peptides Analysis, Synthesis, Biology, supra.

Another possibility is the application of enzymes in peptides synthesis; for reviews see e.g. H.D. Jakubke in The Peptides, Analysis, Synthesis Biology, Vol. 9, S. Udenfriend and J. Meienhofer, eds., (Acad. Press, N.Y., 1987).

### III. Diagnostic Methods-

In a preferred diagnostic method according to the invention, labelled (e.g. with a fluorescent compound) antibodies directed against the extracellular prosomes are used to identify or isolate extracellular prosomes in a patient's blood.. The amount of prosomes thus labelled are measured or counted, and the measurement is compared with a norm or standard measurement.

Several devices can be used with the method.

As used herein an "immunochemical reagent" means that the antibodies displaying the appropriate selectivity have been bound to a suitable support or have been provided with a labelling substance. Supports typically used include the inner wall of a microtest well or a cuvette, a tube or capillary, a membrane filter, test strip, or the surface of a particle such as a latex particle, bead, erythrocyte, dye sol, metal (e.g. gold) sol or metal compound as sol particle. Labelling substances typically used include various radioactive isotopes, fluorescent compounds, enzymes, dye sols or metal compounds used as sol particles.

In one method of detecting the presence of extra-cellular prosomes, an immunochemical reagent containing the anti-prosomal protein antibodies is brought into contact with the test fluid. After which the presence of immune complexes formed between the antibodies and the prosomes in the test fluid is measured for the presence of an immunochemical reaction. The immunochemical reaction is preferably a sandwich reaction (using labelled antibody), an agglutination reaction (using also sols), a competition reaction, or an inhibition reaction.

In one type of immunoassay, antibodies directed against the cell or cells to be studied are attached to a solid phase, for example the inside of a test tube. The bodily fluid containing the portion of blood which would contain the extracellular prosomes is are then added to the tube so as to bind with the antibody. To the tube coated with the antigen-antibody complex is added an immunological reagent containing a known amount of the appropriate anti-prosomal protein antibody or antibodies labelled with a fluorescent compound. The amount of fluorescence is thus measured, and compared with a norm.

Other diagnostic test kits are known to those skilled in the art which may be adapted for use in the instant invention. For example, immunological diagnostic test kits include radio-immunoassay or enzyme immunoactivity assay ("EIA") as described in U.S. Patent Reissue No. 32,696 to Schuurs et al. A light scattering immunoassay system is disclosed in EP 0,254,430 A2 to Ortho Diagnostic Systems, Inc. (corresponding to U.S. Serial No. 879,236 filed 26 June 1986). Diagnostic test kits using two monoclonal antibodies of high binding affinity are described in U. S. Patent Nos. 4,376,110 and 4,486,530 to Hybritech Inc.

### III. Protease activity-

The amount of extracellular prosomes can also be determined by measuring the amount of protease activity in an extracted portion of blood which would contain these extracellular prosomes. The greater the number of prosomes the greater the amount of protease activity.

The amount of protease activity in the pool can be measured using the following substrate: 5 microliters digested 0.22 pM of substrate in 30 minutes (see FIG. D). MCA represents 4-methyl-7-cumarylamido.

The invention is further explained by reference to the following illustrative example.

### EXAMPLE I

Human or calf sera were centrifuged at 3000 RPM and then at 12,000 RPM to spin down any dead cells or remaining debris. The clarified supernatant was then centrifuged through a sucrose cushion 18 hours at 37,000 RPM in a Beckman Ti60 rotor to collect the particles. The pellet was resuspended in a buffer containing 0.5% Sarkosyl at 50,000 RPM for 5 hours in a Beckman T170 rotor. The resuspended pellet was analysed by polyacrylamide gel electrophoresis ("PAGE") and Western blot with anti-prosome monoclonal antibodies revealing the presence of prosomal antigens. When loaded onto a 5-21% sucrose gradient, intact prosomes were observed in the 19S region (panel A of FIG. 1). Prosomal proteins were revealed in each fraction of the sucrose gradient by Western blot using a panel of anti-prosome monoclonal antibodies (anti-p25K, anti-p27K, anti-p29K, anti-p31K, and anti-p33K) and secondary anti-mouse peroxidase conjugated antibody (panel C of FIG. 1, MW markers are shown on the right hand side). These extracellular prosomes contain pRNA's in the 70 nucleotide range (panel B), as revealed by 3' labelling and acrylamide-urea gel electrophoresis.

Protease assays were performed showing that these prosomes possess multicatalytic protease activity:

### PROTEASE ACTIVITY IN POOL (5 µl digested 0.22 pM of substrate Leu-Leu-Val-Tyr-MCA in 30 minutes)

| | | |
|---|---|---|
| Amount | µl | 5 |
| Fluorescence measured | u | 39 |
| Liberated substrate | pM | 0.22 |

### EXAMPLE II (Comparative example)

Extra-cellular prosomes were prepared from sera obtained from normal subjects, and from patients suffering from colon cancer, by differential centrifugation in presence of sarkosyl as described in the EXAMPLE I. Pellets were resuspended in sample buffer (2% B-mercaptoethanol, 2% SDS, 0.0 M Tris-Cl pH 6.8, 12% saccharose and bromophenol blue) and analysed by polyacrylamide gel electrophoresis followed by transfer onto nitrocellulose membrane and probing with a mixture of the anti-prosome monoclonal antibodies: anti-p27K (Ib5) and anti-p25K (7A11). The fixed anti-prosome monoclonal antibodies were revealed by rabbit anti-mouse IgG antibodies coupled to peroxidase and developed by standard reagent (0.02% chloro-napthol in methanol phosphate buffered saline 20/80 containing 1/1000 volume H₂O₂). Each column of FIG. 2 corresponds to extra-cellular prosomes purified from 4 ml sera:
- (1 and 2):: sera from patients afflicted with colon cancer; and
- (3 and 4)):: sera from control patients.

The use of specific examples and embodiments should not be construed as limitations to the scope of the invention which is defined by the appended claims.

## Claims

1. An in vitro method for the diagnosis of human immunodeficiency virus infection in a subject comprising:
quantitatively determining the amount or percentage of extracellular prosomes present in the subject's blood or fraction thereof; and
comparing the amount or percentage thus obtained with a standard from a person not infected with human immunodefiency virus.

2. The method of claim 1 wherein the method of quantitatively determining the amount or percentage of extracellular prosomes comprises analyzing, with labelled antibodies, labelled antibody fragments or mixtures thereof, a sample of the subject's blood or extract thereof.

3. The method of claim 2 wherein the antibodies or antibody fragments are selected or derived from the group of antibodies directed against prosomal proteins p25K, p27K, p29K (p28K,p33K), p30/33K, and p31K.

4. The method of claim 1 wherein the method of obtaining a quantitative determination of extracellular prosomes analyzing the subject's blood or fraction thereof, with the substrate: to determine the amount of protease activity of any of said extracellular prosomes.

5. A method of using the substrate: for manufacturing a diagnostic test kit for the diagnosis of human immunodeficiency virus.

## Patentansprüche

1. In vitro-Verfahren zur Diagnose einer Infektion mit dem humanen Immundefizienz-Virus bei einem Patienten, umfassend:
- quantitative Bestimmung der Menge oder des Prozentsatzes an im Blut des Patienten oder in Fraktionen davon vorhandenen extrazellulären Prosomen, und
- Vergleich der so erhaltenen Anzahl oder des Prozentsatzes mit dem Standard-Wert einer nicht mit dem humanen Immundefizienz-Virus infizierten Person.

2. Verfahren gemäss Anspruch 1, wobei das Verfahren zur quantitativen Bestimmung der Menge oder des Prozentsatzes an extrazellulären Prosomen die Analyse des Bluts des Patienten oder von Extrakten daraus mittels markierter Antikörper, markierter Antikörper-Fragmente oder daraus hergestellter Mischungen umfasst.

3. Verfahren gemäss Anspruch 2, wobei die Antikörper oder Antikörper-Fragmente aus einer aus gegen die Prosomenproteine p25K, p27K, p29K (p28K, p33K), p30/33K und p31K gerichteten Antikörpern bestehenden Gruppe ausgewählt oder davon abgeleitet werden.

4. Verfahren gemäss Anspruch 1, wobei das Verfahren zum Erhalten einer quantitativen Bestimmung extrazellulärer Prosomen die Analyse des Bluts des Patienten oder von Fraktionen davon mittels des Substrats: zur Bestimmung der Proteaseaktivität eines der genannten extrazellulären Prosomen umfasst.

5. Verfahren zur Verwendung des Substrats: zur Herstellung eines Diagnose-Testsets zur Diagnose des humanen Immundefizienz-Virus.

## Revendications

1. Une méthode in vitro de diagnostic d'une infection par le virus de l'immunodéficience humaine chez un sujet comprenant :
le dosage quantitatif de la quantité ou du pourcentage de prosomes extracellulaires présents dans le sang du sujet ou dans une fraction du sang et,
la comparaison de la quantité ou du pourcentage ainsi obtenue avec une référence obtenue à partir d'un sujet non infecté par le virus de l'immunodéficience humaine.

2. La méthode selon la revendication 1 dans laquelle la méthode de dosage quantitatif de la quantité ou du pourcentage de prosomes extracellulaires comprend l'analyse, à l'aide d'anticorps marqués, de fragments d'anticorps marqués ou de leurs mélanges, d'un échantillon du sang ou d'un extrait de sang du sujet.

3. La méthode selon la revendication 2 dans laquelle l'anticorps ou les fragments d'anticorps sont choisis ou dérivés du groupe d'anticorps dirigés contre des protéines prosomales p25k, p27k, p29k (p28k, p33k) , p30/33k et p31k.

4. La méthode selon la revendication 1 dans laquelle la méthode d'obtention d'un dosage quantitatif des prosomes extracellulaires comprend l'analyse du sang ou d'une fraction de sang d'un sujet avec le substrat : pour déterminer l'activité de protéase de n'importe lequel desdits prosomes extracellulaires.

5. Une méthode d'utilisation du substrat pour la préparation d'un kit de test de diagnostic pour le diagnostic de la présence du virus de l'immunodéficience humaine.
